# EUROPÄISCHE PATENTANMELDUNG

(11) **EP 4 792 760 A1**
(43) Veröffentlichungstag der Anmeldung: **19.08.2026**
(21) Anmeldenummer: 25157486.9
(22) Anmeldetag: 12.02.2025
(51) Int. Cl.: A61B 17/80, A61B 17/88

(54) **KNOCHENPLATTE UND SYSTEM ZUM BEHANDELN EINER FRAKTUR EINER CLAVICULA UND/ODER VON ABRISSEN VON CORACO-CLAVICULÄREN, CORACO-ACROMIALEN, UND/ODER ACROMIO-CLAVICULÄREN BÄNDERN**

(71) Anmelder: Medartis AG, 4057 Basel (CH)
(72) Erfinder: ZEUNER, Hermann, 79111 Freiburg (DE)
(74) Vertreter: Hepp Wenger Ryffel AG

(57) **Zusammenfassung**

Die Erfindung betrifft eine Knochenplatte (10) zum Behandeln einer Fraktur einer Clavicula (50) und/oder von Abrissen der coraco-claviculären (D3, D4), coraco-acromialen (D2), und/oder acromio-claviculären (D1) Bänder. Die Knochenplatte (10) umfasst einen Clavicula-Abschnitt (11) zum Anlegen an die Clavicula (50), wobei der Clavicula-Abschnitt (11) einen lateralen Endabschnitt (13), einen medialen Endabschnitt (18), einen lateralen Rand (22), einen posterioren Rand (21), einen medialen Rand, einen anterioren Rand (14), eine superiore Oberfläche, und eine inferiore Oberfläche sowie mindestens eine Öffnung (12) zum Einsetzen eines Fixierelements aufweist. Die Knochenplatte (10) enthält einen Acromion-Abschnitt (16) zum Anlegen an ein der Clavicula (50) benachbartes Acromion (51) und einen Zwischenabschnitt (15), welcher sich vom Clavicula-Abschnitt (11) zum Acromion-Abschnitt (16) erstreckt. Erfindungsgemäss erstreckt sich der Zwischenabschnitt (15) in superiorer Ansicht seitlich, von einer Verbindungsstelle des Clavicula-Abschnitts (11) weg. Die Verbindungsstelle befindet sich wenigstens teilweise am posterioren oder am anterioren Rand.

## Beschreibung

Die Erfindung betrifft eine Knochenplatte, ein System, und ein Verfahren zum Behandeln einer Fraktur einer Clavicula und/oder von Abrissen von coraco- und/oder acromio-claviculären Bändern gemäss dem Oberbegriff der unabhängigen Ansprüche.

Aus dem Stand der Technik sind implantierbare Platten zur Behandlung einer Fraktur und/oder von Bänderabrissen der Clavicula bekannt.

US 2011/0184414 A1 offenbart eine Knochenfixationsplatte, die so geformt ist, dass sie sich der Anatomie des Schlüsselbeins anpasst, und einen lateralen Bereich umfasst, der sich zum Acromion hin erstreckt.

US 2023/0071963 A1 offenbart eine Knochenplatte zur Behandlung einer acromio-claviculären Dislokationen mit einem Hakenelement, welches sich unter das Acromion erstreckt.

WO 2018/222449 A1 offenbart eine Knochenplatte zur Behandlung von acromio-clavikulären Dislokationen mit einem Hakenelement.

Die Knochenplatten des Stands der Technik weisen oftmals einen Haken auf, der durch eine anatomische Lücke im postero-lateralen Bereich der Clavicula durch das Acromio-Claviculargelenk geführt wird. Die Knochenplatte wird daraufhin typischerweise auf der Clavicula fixiert, etwa durch Knochenschrauben. Dadurch kann die Clavicula heruntergedrückt werden, während gleichzeitig das Acromion, etwa von inferior in superior Richtung, angehoben und/oder abgestützt wird. Dadurch wird während der Heilungsphase der Fraktur und/oder der Bänder eine physiologisch korrekte Ausrichtung der Lage des Acromion und der Clavicula zueinander erreicht.

Die Clavicula und das Acromion sind durch das Ligamentum acromio-claviculare verbunden. Zudem ist die Clavicula mit dem Coracoideus durch das Ligamentum coraco-claviculare verbunden. Das Ligamentum trapezoideum und Ligamentum conoideum bilden das Ligamentum coraco-claviculare. Das Ligamentum trapezoideum ist lateral positioniert und weist eine eher horizontale Ausrichtung auf. Die horizontale Ausrichtung erstreckt sich im entlang der anterior-posterioren und lateral-medialen Richtung. Das Ligamentum conoideum weist eine vertikalere Ausrichtung auf. Die vertikalere Ausrichtung erstreckt sich eher entlang der inferior-superioren Richtung. Das Acromio-Claviculargelenk befindet sich zwischen der Clavicula und dem Acromion.

Die bekannten Knochenplatten, Systeme, und Verfahren zum Behandeln einer solchen Fraktur und/oder eines solchen Abriss weisen jedoch verschiedene Nachteile auf. Insbesondere wenn die Knochenplatte das Acromion von inferior in superiorer Richtung stützt und durch einen Acromio-Claviculargelenkspalt geführt wird, ist die Fixierung der Knochenplatte erschwert. Durch eine Platzierung des Hakens der Platte, der durch eine anatomische Lücke im postero-lateralen Bereich der Clavicula durch das Acromio-Claviculargelenk geführt werden soll, kann oftmals keine passgenaue Ausrichtung von Acromion und Clavicula zueinander in superior-inferiorer Richtung ermöglicht werden. Ausserdem kann die Funktion des Gelenks durch die Platte oder Teile der Platte eingeschränkt werden. Zudem kann durch Anbringung der Platte eine erhöhte mechanische Belastung auf die Anatomie des Patienten ausgeübt werden. Durch die implantierte Knochenplatte werden das Acromion, die Clavicula, und insbesondere das Acromio-Claviculargelenk oftmals intra- und postoperativ belastet. Eine solche mechanische Belastung kann etwa Schultereckgelenksarthrose (ACG-Arthrose) fördern und/oder postoperative Schmerzen verursachen. Die Fixierung einer solchen Knochenplatte kann während und nach der Implantation zu Spannungen und mechanischen Belastungen zwischen dem Haken der Knochenplatte und dem Acromion führen, was etwa einen erhöhten Knochenabrieb zur Folge haben kann. Zudem ist durch die Positionierung des Hakens in der anatomischen Lücke eine medial-laterale Positionierbarkeit der Knochenplatte oftmals eingeschränkt.

Es ist daher die Aufgabe der vorliegenden Erfindung, die Nachteile des Bekannten zu vermeiden und insbesondere eine Knochenplatte, und ein Verfahren bereitzustellen, sodass die Knochenplatte einfach implantiert werden kann, eine stabile und sichere Fixierung sowie die physiologisch richtige Lage des Acromion in Relation zur Clavicula in Richtung superior-inferior ermöglicht wird, und Anwendungsfehler reduziert werden. Zudem soll eine optimierte Anpassung und Anlegbarkeit an die Anatomie des Patienten ermöglicht werden und eine kostengünstige sowie einfache Anwendung mit kurzer Lernkurve von Klinikern erzielt werden.

Erfindungsgemäss werden diese und andere Aufgaben mit einer Knochenplatte, einem System umfassend die mindestens eine Knochenplatte sowie mindestens ein plattenspezifisches Biegewerkzeug, und einem Verfahren zum Behandeln einer Fraktur einer Clavicula und/oder von Abrissen von coraco-claviculären, coraco-acromialen, und/oder acromio-claviculären Bändern mit den Merkmalen der unabhängigen Ansprüche gelöst.

Die erfindungsgemässe Knochenplatte ist zum Behandeln einer Fraktur einer Clavicula und/oder von Abrissen der coraco-claviculären, coraco-acromialen, und/oder acromio-claviculären Bänder geeignet. Die Knochenplatte enthält einen Clavicula-Abschnitt zum Anlegen an die Clavicula. Der Clavicula-Abschnitt weist einen lateralen Endabschnitt, einen medialen Endabschnitt, einen lateralen Rand, einen posterioren Rand, einen medialen Rand einen anterioren Rand eine inferiore Oberfläche und eine superiore Oberfläche auf, sowie mindestens eine Öffnung zum Einsetzen eines Fixierelements auf. Das Fixierelement kann eine Knochenschraube sein. Die Knochenplatte umfasst einen Acromion-Abschnitt zum Anlegen an ein der Clavicula benachbartes Acromion. Zudem enthält die Knochenplatte einen Zwischenabschnitt, welcher sich vom Clavicula-Abschnitt zum Acromion-Abschnitt erstreckt.

Der Clavicula-Abschnitt kann eine Längsachse aufweisen, die kurvilinear ausgebildet sein kann und insbesondere im Bereich des medialen Endabschnitts im Wesentlichen gerade verläuft. Der Acromion-Abschnitt kann eine weitere kurvilineare Längsachse aufweisen.

Der Zwischenabschnitt kann sich erfindungsgemäss insbesondere in superiorer Ansicht seitlich von einer Verbindungsstelle vom Clavicula-Abschnitt weg erstrecken. Die Verbindungsstelle kann sich wenigstens teilweise am posterioren oder am anterioren Rand des Clavicula-Abschnitts befinden.

Alternativ oder zusätzlich kann die Form und Grösse des Zwischenabschnitts und des Acromion-Abschnitts erfindungsgemäss derart gewählt werden, dass bei bestimmungsgemässer Anwendung der Zwischenabschnitt, und insbesondere alle Bestandteile der Platte, zumindest teilweise und bevorzugt vollständig ausserhalb des Acromio-Claviculargelenkspalts liegen.

Alternativ oder zusätzlich kann die Form und Grösse des Zwischenabschnitts erfindungsgemäss derart gewählt werden, dass bei bestimmungsgemässer Anwendung mindestens ein Teil des Zwischenabschnitts sich um eine posteriore oder um eine anteriore Seite am lateralen Ende der Clavicula herum erstreckt.

Die anatomischen Raumrichtungen des Patienten, nämlich mediallateral, anterior-posterior und superior-inferior, entsprechen den Raumrichtungen einer ordnungsgemäss implantierten Position der Knochenplatte.

Ein solcher Zwischenabschnitt ermöglicht eine besonders einfache Herstellung, minimiert Interaktionen mit Weichteilen und insbesondere den Gelenkspalt begrenzenden Gelenkflächen und/oder den Bändern, reduziert mechanisch bedingte Irritationen und Wechselwirkungen, und fördert eine optimale biomechanische Integration der Knochenplatte. Zudem ermöglicht dies, die Knochenplatte an unterschiedlichen medial-lateralen Positionen relativ zur Clavicula zu positionieren, da der Zwischenabschnitt nicht angrenzend an eine laterale Endfläche der Clavicula angeordnet werden muss.

Der Zwischenabschnitt kann sich in superiorer Ansicht seitlich von der Verbindungsstelle erstrecken, sodass der Zwischenabschnitt sich mindestens teilweise in anterior-posteriorer Richtung erstreckt.

Der Zwischenabschnitt kann sich in einer lateralen Ansicht der Knochenplatte auch zumindest teilweise in superior-inferiorer Richtung erstrecken.

Der Zwischenbereich kann sich sowohl in anterior-posteriorer Richtung als auch in superior-inferiorer Richtung seitlich vom posterioren oder anterioren Rand weg erstrecken.

Dabei erstreckt sich der Zwischenabschnitt nicht ausschliesslich und bevorzugt auch nicht teilweise vom lateralen Rand weg.

Der Zwischenabschnitt weist dabei insbesondere eine Breite in medial-lateraler Richtung auf, die grösser ist als seine Dicke in superior-inferiorer Richtung an der Verbindungsstelle. Der Zwischenabschnitt kann sich in einer superioren/inferioren Blickrichtung mindestens teilweise parallel zur Längsachse des lateralen Endabschnitts erstrecken. Vorzugsweise erstreckt sich der Zwischenabschnitt in der superioren/inferioren Blickrichtung mindestens teilweise parallel zur Längsachse des lateralen Endabschnitts.

Alternativ kann der Zwischenabschnitt sich zumindest teilweise in der superioren/inferioren Blickrichtung in einem Winkel von - 30° bis 30°, insbesondere -10° bis 10°, vorzugsweise -5° bis 5°, zur Längsachse des lateralen Endabschnitts erstrecken. Durch eine solche Knochenplatte lässt sich der Kontakt der Knochenplatte, insbesondere des Zwischenabschnitts und des Acromion-Abschnitts, mit dem Acromio-Claviculargelenk minimieren oder sogar ganz verhindern. Insbesondere kann eine mechanische Beanspruchung des Ligamentum acromio-claviculare, des Ligamentum coraco-acromiale, des Ligamentum trapezoideum, und des Ligamentum conoideum vermieden oder reduziert werden.

Die Knochenplatte kann bei einem Abriss eines der coraco-claviculären Bänder, insbesondere des ligamentum trapezoideum und/oder des ligamentum conoideum, und eventueller Fraktur der Clavicula eine temporäre Fixierung ermöglichen. Zugleich kann die Knochenplatte dennoch auf einfache Weise und zuverlässig dafür eingesetzt werden, formkongruent an das Acromion und die Clavicula angelegt zu werden.

Die inferiore Oberfläche des Clavicula-Abschnitts kann teilweise oder vollständig auf der Clavicula anlegbar sein, insbesondere auf einer superioren Seite der Clavicula. Der Clavicula-Abschnitt kann geformt und dimensioniert sein, um zumindest auf einer Oberseite am lateralen Ende der Clavicula anlegbar, und vorzugsweise befestigbar, zu sein. Der Clavicula-Abschnitt kann eine Vielzahl von Öffnungen aufweisen, sodass er auf der superioren Seite der Clavicula und/oder beidseitig einer Fraktur der Clavicula, insbesondere einer Fraktur am lateralen Ende der Clavicula, anlegbar und befestigbar ist.

Die Knochenplatte weist eine Längsachse auf, die sich vom medialen Endabschnitt zum lateralen Endabschnitt erstreckt und sich durch einen mittleren Punkt des lateralen Randes und des medialen Randes erstreckt. Die Längsachse kann kurvilinear sein, d.h. sie kann sowohl in superiorer als auch anteriorer als auch lateraler Ansicht gerade sein oder ganz oder teilweise gekrümmt verlaufen.

Dass der Zwischenabschnitt sich in superiorer Ansicht "seitlich" vom Clavicula-Abschnitt weg erstreckt, bezieht sich auf die Plattengeometrie, d.h. der Zwischenabschnitt erstreckt sich zumindest teilweise in superiorer Ansicht seitlich weg bezogen auf die Längsachse des Clavicula-Abschnitts. Der Zwischenabschnitt kann sich somit zumindest teilweise seitlich vom anterioren oder posterioren Rand des Clavicula-Abschnitts in einer anterior-posterioren Ebene weg erstrecken. Wenn der Zwischenabschnitt sich vom anterioren oder posterioren Rand in superiorer Ansicht seitlich weg erstreckt, insbesondere vom posterioren Rand, kann eine reduzierte Interaktion mit den umliegenden Weichteilen, der Gelenkkapsel, den Bändern und der Nerven erzielt werden.

Abgesehen vom sich seitlich weg erstreckenden Bereich des Zwischenabschnitts kann der Zwischenabschnitt sich zumindest teilweise entlang einer superioren-inferioren Richtung erstrecken.

Der posteriore Rand und/oder der anteriore Rand des Clavicula-Abschnitts können einen Übergangsbereich zum lateralen Rand umfassen. Der Zwischenabschnitt kann sich auch ganz oder teilweise von einem solchen Übergangsbereich weg erstrecken. Der Übergangsbereich kann z.B. eine Ecke oder einen abgerundeten Bereich zwischen dem anterioren oder posterioren Rand und dem lateralen Rand bilden. Die Verbindungsstelle, von der sich der Zwischenabschnitt erstreckt, kann sich aber auch vollständig ausserhalb des Übergangsbereichs am anterioren oder posterioren Rand befinden.

Die Form und Grösse des Zwischenabschnitts und des Acromion-Abschnitts kann derart gewählt sein, dass bei bestimmungsgemässer Anwendung der Acromion-Abschnitt das Acromion von inferior in Richtung superior unterstützt und sich teilweise um das Acromion erstreckt.

Der Zwischenabschnitt kann ganz oder teilweise gebogen und an eine äussere Kontur der posterioren oder anterioren Aussenfläche der Clavicula angepasst sein, sodass ein verbessertes Anliegen des Zwischenabschnitts im Bereich der superior-posterioren bzw. superior-anterioren Kante des Knochens erzielt werden kann.

Damit kann das Acromion zuverlässig, insbesondere von inferior, abgestützt werden, um eine optimale Ausrichtung von Acromion und Clavicula wiederherzustellen, insbesondere ohne das Acromion starr relativ zur Clavicula zu fixieren. Der Acromion-Abschnitt kann loch-/öffnungsfrei sein, sodass keine Fixierelemente darin anbringbar sind.

Der Zwischenabschnitt kann bezogen auf eine posterior-anteriore Richtung zumindest teilweise eine Krümmung in einer lateralen Seitenansicht aufweisen.

Die Krümmung kann so gewählt sein, dass ein mittlerer Abstand des Acromion-Abschnitts von dem mittleren Punkt des lateralen Randes in posterior-anteriorer Richtung in einer der lateralen Ansicht kleiner ist, als der mittlere Abstand des Zwischenabschnitts von dem mittleren Punkt. Der mittlere Abstand des Acromion-Abschnitts zum mittleren Punkt kann in einem Bereich von - 20 mm bis 20 mm, insbesondere -10 mm bis 15 mm, vorzugsweise - 5 bis 10 mm, liegen.

Die Krümmung kann kontinuierlich sein oder es können einzelne gebogene Bereiche entlang von Biegekanten vorliegen.

Dies ermöglicht, dass der Acromion-Abschnitt eng an das Acromion anlegbar ist. Zugleich kann der Zwischenabschnitt schonender angeordnet werden, insbesondere ohne das Acromio-Claviculargelenk zu sehr zu belasten, sodass die Regeneration und biomechanische Funktion des Gelenks verbessert werden.

In superiorer Ansicht kann der laterale Rand des Clavicula-Abschnitts zumindest teilweise parallel zur anterioren-posterioren Richtung verlaufen, und insbesondere abgerundete Ecken aufweisen.

Der Acromion-Abschnitt kann zumindest abschnittsweise eine oder mehrere Krümmungen aufweisen. Zumindest eine Krümmung kann sich in einer superioren Ansicht in anterior-posteriorer Richtung erstrecken. Zumindest eine Krümmung kann sich in einer anterioren Ansicht in superior-inferiorer Richtung erstrecken.

Eine solche Krümmung des Acromion-Abschnitts ermöglicht, dass der Acromion-Abschnitt einfach an das Acromion anlegbar und anpassbar ist.

Die Längsachse des Acromion-Abschnitts kann in einer posterioren Ansicht relativ zu der Längsachse im lateralen Endabschnitt des Clavicula-Abschnitts einen Winkel oder Winkelbereich ausgewählt aus 1° bis 60° aufweisen, insbesondere 5° bis 50°, vorzugsweise 10° bis 45°.

Die Längsachse des Acromion-Abschnitts kann in einer superioren Ansicht relativ zu der Längsachse im lateralen Endabschnitt des Clavicula-Abschnitts einen Winkel oder Winkelbereich ausgewählt aus 1° bis 60° aufweisen, insbesondere 5° bis 50°, vorzugsweise 10° bis 45°.

Die Breite in anterior-posteriorer Richtung und/oder die Dicke in superior-inferior Richtung des Acromion-Abschnitts kann sich ausgehend von dem Zwischenabschnitt verändern. Insbesondere kann sie sich kontinuierlich oder abschnittsweise verjüngen oder verbreitern.

Dies ermöglicht eine optimierte Kontaktierung des Acromion mit dem Acromion-Abschnitt.

Der Acromion-Abschnitt kann zudem eine sich verjüngende, aber atraumatisch abgerundete, Spitze aufweisen, sodass das Acromion leichter angehoben werden kann und die Knochenplatte besser zum Anheben des Acromion in lateraler Richtung vorgeschoben werden kann.

Die Form und Grösse des Acromion-Abschnitts kann in Abhängigkeit des Materials derart gewählt sein, dass der Acromion-Abschnitt und/oder der Zwischenabschnitt manuell mit einem plattenspezifischen Biegewerkzeug biegbar und/oder um seine Längsachse tordierbar ist. Das Material ist insbesondere Titan, eine Titanlegierung, ein Implantatstahl oder eine Metalllegierung.

Dies ermöglicht, dass der Acromion-Abschnitt pathologie-/patientenspezifisch manuell angepasst werden kann, um eine optimierte Behandlung zu ermöglichen.

Der Acromion-Abschnitt der Knochenplatte kann in einer Seitenansicht in lateral-medialer Richtung im Wesentlichen U-, C-, I-, J- oder Z-förmig ausgebildet sein.

Der Zwischenabschnitt kann in einer Seitenansicht in lateral-medialer Richtung im Wesentlichen U-, C-, I-, J- oder Z-förmig ausgebildet sein.

Solche, insbesondere die U-, C-, I-, J- oder Z-förmigen Acromion-Abschnitte und/oder Zwischenabschnitte sind fertigungstechnisch leicht und effizient herzustellen. Ein solcher, etwa C- oder U-förmiger, Acromion-Abschnitt kann so dimensioniert und geformt sein, dass das Acromion besonders zuverlässig von inferior in superiorer Richtung kontaktiert werden kann.

Ein etwa C- oder U-förmiger Zwischenabschnitt kann so dimensioniert und geformt sein, dass er sich teilweise um die Clavicula erstreckt. Somit kann der Zwischenabschnitt in lateral-medialer Richtung leichter umpositioniert werden und/oder sich eng an der Knochenanatomie entlang erstrecken.

Bevorzugt wird die Knochenplatte durch Umformen eines gefrästen Rohlings hergestellt. Alternativ ist auch eine Fertigung aus dem Vollen, z.B. durch Fräsen der Platte, oder durch additive Fertigung möglich.

Der Acromion-Abschnitt kann zum mittleren Punkt des lateralen Randes des Clavicula-Abschnitts in einer superioren Ansicht auf den lateralen Endabschnitt einen minimalen Abstand aufweisen, der im Bereich von 0.1 mm bis 35 mm, insbesondere 1 mm bis 25 mm, vorzugsweise 1.5 mm bis 15 mm, liegt. Alternativ kann der Abstand im Bereich von 4 mm bis 13 mm, insbesondere 5.5 mm bis 11.5 mm, vorzugsweise 7 mm bis 10 mm liegen. Damit wird erreicht, dass bei der Implantation der Clavicula-Abschnitt auf dem lateralen Teil der Clavicula liegt, ohne dass der Acromion-Abschnitt die Clavicula von inferior berührt. Ansonsten könnte die Knochenplatte bei Claviculae mit dickem Knochen gar nicht angelegt werden könnte, weil der Acromion Abschnitt den Knochen einklemmt, oder falsch dimensioniert ist um sich um den Knochen herum zu erstrecken.

Ein laterales Ende des Clavicula-Abschnitt kann mindestens teilweise in einer Claviculaebene liegen. Die Claviculaebene und die Längsachse des Acromion-Abschnitts können in einer posterioren Ansicht unter einem Winkel oder Winkelbereich von 0.1° bis 45°, insbesondere 3° bis 35°, vorzugsweise 6° bis 25°, zueinander verlaufen.

Der gesamte Acromion-Abschnitt kann eine superiore-inferiore Ausdehnung von 2 mm bis 25 mm, insbesondere 3 mm bis 15 mm, vorzugsweise von 4 mm bis 10 mm, aufweisen.

Der gesamte Acromion-Abschnitt kann eine posteriore-anteriore Ausdehnung von 2 mm bis 25 mm, insbesondere 3 mm bis 15 mm, vorzugsweise von 4 mm bis 10 mm, aufweisen.

Der gesamte Acromion-Abschnitt kann eine laterale-mediale Ausdehnung von 5 mm bis 50 mm, insbesondere 10 mm bis 40 mm, vorzugsweise von 15 mm bis 30 mm, aufweisen.

Der Acromion-Abschnitt kann diese Ausdehnungen in Kombination aufweisen.

Damit kann eine Anhebung des Acromions in superiorer Richtung einfach erzielt werden und der Clavicula-Abschnitt kann anliegend auf der Clavicula positioniert werden. Zudem kann durch eine laterale oder mediale Verschiebung der Knochenplatte relativ zur Clavicula die Positionierung des Acromions und somit ein Abstand in superiorer-inferiorer Richtung relativ zur Clavicula gezielt angepasst werden. Je nachdem, wie weit die Knochenplatte lateral oder medial vorgeschoben wird, kann das Acromion patientenspezifisch gemäss der jeweiligen Physiologie weiter angehoben oder abgesenkt werden. Da der Zwischenabschnitt sich in superiorer Ansicht seitlich vom anterioren oder posterioren Rand erstreckt und nicht ausschliesslich in der anatomischen Lücke im posterioren-lateralen Bereich der Clavicula anliegt, kann der Clavicula-Abschnitts auf der Clavicula einfach verschoben werden und seine Position in lateraler oder medialer Richtung leichter variiert werden.

Ein in einer Ansicht von lateral senkrecht zum Clavicula-Abschnitt gemessener Abstand des Acromion-Abschnitts vom Clavicula-Abschnitt kann in einem Bereich von 0.1 mm bis 30 mm liegen, insbesondere in einem Bereich von 2 mm bis 25 mm, vorzugsweise in einem Bereich von 5 mm bis 18 mm.

Damit kann die Knochenplatte auf der superioren Oberfläche der Clavicula platzierbar sein und gleichzeitig das Acromion in superiorer Richtung stützen.

Der Clavicula-Abschnitt kann vom lateralen Rand zum medialen Rand in Richtung der Längsachse in einer Ansicht von superior eine erste Länge von 15 mm bis 180 mm, insbesondere von 30 mm bis 110 mm, vorzugsweise von 40 mm bis 100 mm aufweisen. Der Acromion-Abschnitt kann von einem medialen Ende zu einem lateralen Ende entlang einer Längsachse in einer Ansicht von superior eine zweite Länge von 5 mm bis 40 mm, insbesondere von 10 mm bis 35 mm, vorzugsweise von 20 mm bis 30 mm, aufweisen.

Eine superiore und/oder inferiore Oberfläche des Acromion-Abschnitts kann gewölbt sein, insbesondere bombiert in superiorer Richtung.

Die Längsachse des Acromion-Abschnitts kann in einer superioren Ansicht unter einem Winkel relativ zur Längsachse durch den lateralen Endabschnitt des Clavicula-Abschnitts verlaufen. Der Winkel oder der Winkelbereich kann im Bereich von -60° bis 60°, insbesondere -30° bis 30°, vorzugsweise -15° bis 15°, liegen.

Die Dimensionierung und Formgebung der Knochenplatte ermöglichen eine Platzierung des Clavicula-Abschnitts und des Acromion-Abschnitts beabstandet vom Acromio-Claviculargelenk, während das Acromion dennoch zuverlässig gestützt werden kann.

Der Zwischenabschnitt kann zumindest einen gebogenen Bereich aufweisen, sodass der Zwischenabschnitt in einer lateralen Seitenansicht in anterior-posteriorer Richtung um einen Winkel von 90° bis 200°, insbesondere um 120° bis 190°, vorzugsweise um 160° bis 185°, gebogen ist.

Der Zwischenabschnitt kann eine laterale-mediale Ausdehnung von 1 mm bis 50 mm, insbesondere 5 mm bis 40 mm, vorzugsweise von 10 mm bis 30 mm, aufweisen.

Der Zwischenabschnitt kann eine posteriore-anteriore Ausdehnung von 3 mm bis 25 mm, insbesondere 5 mm bis 20 mm, vorzugsweise 7 mm bis 15 mm, aufweisen.

Der Zwischenabschnitt kann eine superiore-inferiore Ausdehnung von 5 mm bis 45 mm, insbesondere 10 mm bis 30 mm, vorzugsweise von 10 mm bis 25 mm, aufweisen.

Der Zwischenabschnitt kann diese Ausdehnungen in Kombination aufweisen.

Der Zwischenabschnitt kann in Ansichten von superior und/oder lateral und/oder posterior einen oder mehrere gekrümmte Bereiche und/oder einen oder mehrere gerade Bereiche aufweisen.

Der Zwischenabschnitt kann sich bei einer Ansicht von superior in lateraler Richtung über eine Breite ausgewählt von 1 mm bis 50 mm, insbesondere 10 mm bis 40 mm, vorzugsweise 20 mm bis 30 mm erstrecken. Der Zwischenabschnitt kann sich in einer anterioren oder posterioren Seitensicht in einem Winkel ausgewählt aus einem Bereich von 10° bis 180°, insbesondere 25° bis 120°, vorzugsweise 40° bis 65°, relativ zur Längsachse des Clavicula-Abschnitts erstrecken.

Der Zwischenabschnitt mit zuvor beschriebenen gebogenen Bereichen und einer solchen Länge kann eine, zumindest teilweise, tordierte geometrische Struktur bilden, die sich in lateraler Richtung erstreckt. Die tordierte Struktur kann unterschiedlich stark gebogene Abschnitte und/oder gerade Abschnitte umfassen. Alternativ zum Tordieren kann ein solcher Zwischenabschnitt durch Fräsverfahren erzielt werden.

Die Knochenplatte kann eine Vielzahl von Öffnungen umfassen. Zumindest eine der Öffnungen, insbesondere zwei der Öffnungen, können ein Langloch sein. Das mindestens eine Langloch kann so angeordnet sein, dass bei teilweiser Anbringung eines Fixierelements im Langloch noch eine Verschiebung der Knochenplatte relativ zum Knochen entlang der Längsachse des Langlochs möglich ist. Zumindest eine der Öffnungen kann eine Verblockungskontur zur Aufnahme einer verblockbaren Knochenschraube als Fixierelement aufweisen. Zumindest eine der Öffnungen kann eine Geometrie zur Aufnahme einer Knochenschraube in unterschiedlichen Winkelstellungen aufweisen, insbesondere sodass die Schraube unter verschiedenen Winkeln in der Öffnung verblockt werden kann. Die Knochenschraube und die Öffnung können geformt und/oder dimensioniert sein, um eine Kontur für eine Verblockung zu erzielen, die im Detail in WO 2004/086990 A1 offenbart wird.

Eine Ausführungsform der Knochenplatte kann einen Clavicula-Abschnitt aufweisen, der zwei verschiedene Krümmungen in superiorer Ansicht aufweist, insbesondere mit zwei verschiedenen Krümmungsradien in anterior-posteriorer Richtung.

Eine erste Krümmung des Clavicula-Abschnitts kann sich in superiorer Ansicht im Uhrzeigersinn und eine zweite Krümmung kann sich entgegen dem Uhrzeigersinn erstrecken.

Ein weiterer Aspekt der Erfindung betrifft ein System umfassend eine zuvor beschriebene Knochenplatte sowie ein plattenspezifisches Biegewerkzeug zum manuellen Verbiegen und/oder Tordieren der Knochenplatte.

Ein solches System ermöglicht eine optimale Anpassung an die Physiologie des Patienten, sodass eine patientenspezifische Anlegbarkeit gewährleistet werden kann.

Das System kann Fixierelemente wie Knochenschrauben zum Anbringen der Platte an der Clavicula umfassen. Das System kann einen Bohrer, ein Tiefenmessgerät, und/oder einen Schraubendreher umfassen.

Ein weiterer Aspekt der Erfindung betrifft ein Verfahren zum Behandeln einer Fraktur einer Clavicula und/oder von Abrissen der coraco-claviculären, coraco-acromialen, und/oder acromio-claviculären Bänder mit einer Knochenplatte, insbesondere mit einer zuvor beschriebenen Knochenplatte. Das Verfahren umfasst das Anlegen des Clavicula-Abschnitts an eine Clavicula, ein Anlegen des Acromion-Abschnitts an ein der Clavicula benachbartes Acromion, sodass das Acromion von inferiorer Richtung superior gestützt wird. Die Knochenplatte wird so angelegt, dass die Knochenplatte mindestens teilweise und bevorzugt vollständig ausserhalb des Acromio-Claviculargelenkspalts liegt.

Das Verfahren kann umfassen, dass die Knochenplatte in lateral-medialer Richtung bewegt wird, um die superiore-inferiore Ausrichtung des Acromions und der Clavicula zueinander einzustellen.

Der Acromion-Abschnitt kann, insbesondere wie zuvor beschrieben, geformt und/oder dimensioniert sein, um eine solche Einstellbarkeit zu ermöglichen.

Eine laterale oder mediale Position der Knochenplatte auf einer superioren Oberfläche der Clavicula kann relativ zu der Clavicula so gewählt werden, dass das Acromion durch den Acromion-Abschnitt relativ zur Clavicula so lange angehoben oder abgesenkt wird, bis eine anatomisch korrekte Ausrichtung der beiden Knochen zueinander erzielt wird.

Der Clavicula-Abschnitt kann an der Clavicula befestigt werden, insbesondere indem zumindest ein Fixierelement, insbesondere eine Knochenschraube, durch eine Öffnung der Knochenplatte an der Clavicula befestigt wird.

Ein weiterer Aspekt der Erfindung richtet sich auf einen Rohling der Knochenplatte, der die zuvor beschrieben Acromion-, Zwischen-, und Clavicula-Abschnitte umfasst.

Bei einem Rohling der Knochenplatte kann, vor einer Verformung, der Zwischenabschnitt und/oder Acromion-Abschnitt, insbesondere die ganze Knochenplatte, vollständig in einer Ebene angeordnet sein, die weder Erhebungen noch Vertiefungen aufweist.

Der Zwischenabschnitt und der Acromion-Abschnitt erstrecken sich bei einem solchen Rohling seitlich von der Verbindungsstelle am posterioren oder anterioren Rand des Clavicula-Abschnitt weg, also in posteriorer oder anteriorer Richtung.

Der Zwischenabschnitt und der Acromion-Abschnitt dieses Rohlings können in der Draufsicht gesehen von der Verbindungsstelle bis zum Ende des Acromion Abschnitts gekrümmt verlaufen und insbesondere eine Biegung ausgewählt über einen Winkel von 60° bis 120°, insbesondere 70° bis 100°, vorzugsweise 75° bis 90°, aufweisen. In superiorer Draufsicht erstreckt sich dieser Winkel zwischen einer Tangente eines Längsabschnitt des Zwischenabschnitts unmittelbar angrenzend an die Verbindungsstelle zum posterioren/anterioren Rand und einer Tangente eines terminalen Längsabschnitts des Acromion-Abschnitts.

Bevorzugt ist die Knochenplatte durch Umformen des Rohlings hergestellt, da durch Verbiegung/Tordierung ein geringerer Materialeinsatz und eine einfachere/schnellere Fertigung aus Plattenmaterial erzielt werden kann. Die Knochenplatte wäre ebenfalls durch Fräsen herstellbar. Speziell die Verrundung innenliegender Kanten lassen sich jedoch nur mit erheblichem Mehraufwand beim dreidimensionalen Fräsen herstellen.

Die Erfindung wird nachfolgend anhand der Figuren und Ausführungsbeispiele näher erläutert, die zeigen:
- Figur 1:: eine perspektivische Ansicht einer ersten erfindungsgemässen Knochenplatte,
- Figur 2:: eine Draufsicht aus Richtung superior auf die Knochenplatte nach Fig. 1,
- Figur 3:: eine Draufsicht der erfindungsgemässen Knochenplatte nach Fig. 1 in lateral-medialer Richtung,
- Figur 4:: eine Seitenansicht der erfindungsgemässen Knochenplatte nach Fig. 1 in posterior-anteriorer Richtung,
- Figur 5A:: eine Seitenansicht der erfindungsgemässen Knochenplatte nach Fig. 1 in anteriorer Perspektive, die zur Behandlung einer Fraktur der Clavicula angebracht wurde,
- Figur 5B:: eine Seitenansicht der erfindungsgemässen Knochenplatte nach Fig. 1 in posteriorer Perspektive, die zur Behandlung einer Fraktur der Clavicula angebracht wurde,
- Figur 5C:: eine superiore Draufsicht der erfindungsgemässen Knochenplatte nach Fig. 1, die zur Behandlung einer Fraktur der Clavicula angebracht wurde,
- Figuren 6A: und 6B: eine Draufsicht aus Richtung superior und eine posteriore Seitenansicht einer zweiten, kurzen Ausführungsform der erfindungsgemässen Knochenplatte,
- Figuren 7A: und 7B: eine Draufsicht aus Richtung superior und eine posteriore Seitenansicht einer dritten, mittellangen Ausführungsform der erfindungsgemässen Knochenplatte,
- Figuren 8A: und 8B: eine Draufsicht aus Richtung superior und eine posteriore Seitenansicht einer vierten, langen Ausführungsform der erfindungsgemässen Knochenplatte,
- Figur 9:: eine Draufsicht aus Richtung superior einer fünften erfindungsgemässen Ausführungsform der Knochenplatte,
- Figur 10:: eine Draufsicht aus Richtung superior einer sechsten erfindungsgemässen Ausführungsform der Knochenplatte,
- Figuren 11A und 11B:: eine Draufsicht und Seitenansicht eines plattenspezifischen Biegewerkzeug zum manuellen Verbiegen und/oder Tordieren der Knochenplatte, und
- Figuren 12A und 12B:: eine Draufsicht aus Richtung superior einer erfindungsgemässen Knochenplatte mit einem sich lateral erstreckenden Zwischenabschnitt und Acromion-Abschnitt vor und nach der Verbiegung/Tordierung durch Umformen.

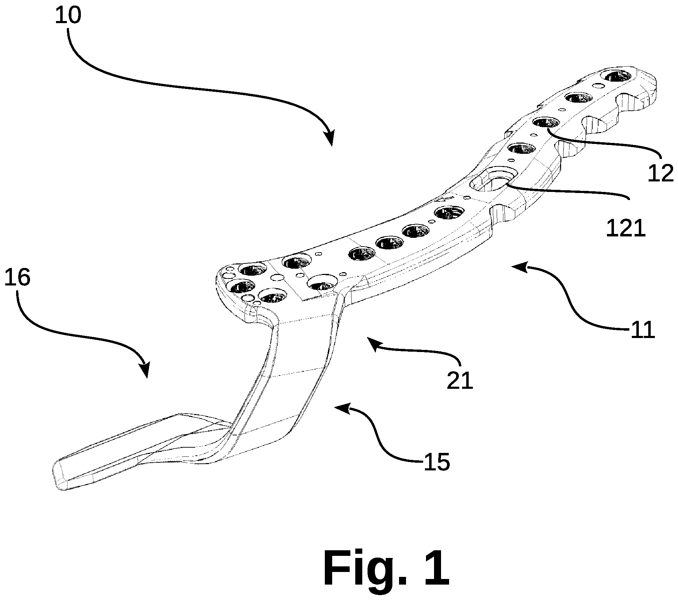

Figur 1 zeigt eine perspektivische Ansicht einer ersten erfindungsgemässen Knochenplatte 10 mit einem Clavicula-Abschnitt 11, einem Zwischenabschnitt 15 und einem Acromion-Abschnitt 16. Der Clavicula-Abschnitt 11 ist entlang zweier verschiedener Krümmungsradien in posterior-anteriorer Richtung gekrümmt und weist eine Vielzahl von Öffnungen 12 zur Aufnahme von Knochenschrauben auf, von denen eine als Langloch 121 ausgebildet ist. Diese gekrümmten Abschnitte verlaufen gegenläufig zueinander. Der Zwischenabschnitt 15 erstreckt sich von einer Verbindungsstelle vom posterioren Rand 21 des Clavicula-Abschnitts 11 in einer Draufsicht aus Richtung superior seitlich weg.

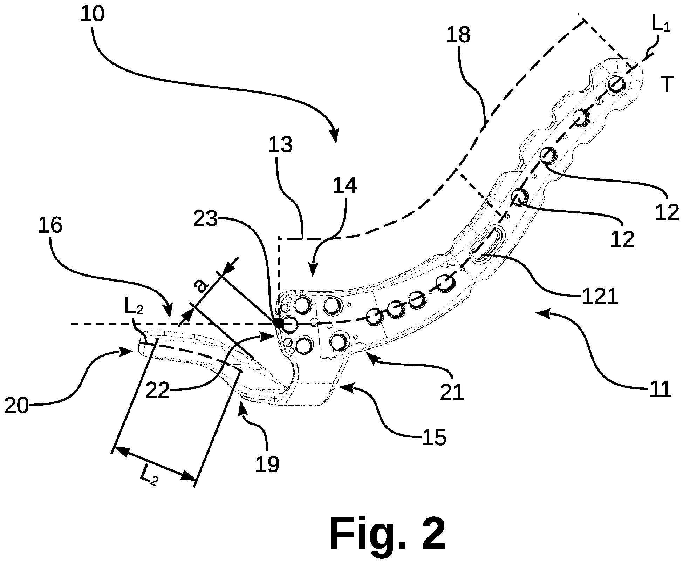

Figur 2 zeigt eine Draufsicht der erfindungsgemässen Knochenplatte 10 nach Fig. 1 aus Richtung superior. Der Clavicula-Abschnitt 11 hat eine kurvilineare Längsachse L₁, die sich entlang der doppelten Krümmung des Clavicula-Abschnitts 11 in medial-lateraler Richtung erstreckt. Der Clavicula-Abschnitt 11 weist einen medialen Endabschnitt 18 auf, welcher sich unter der ersten Krümmung in medial-lateraler Richtung erstreckt. Zudem weist der Clavicula-Abschnitt 11 einen lateralen Endabschnitt 13 auf, welcher sich unter einem zweiten Krümmungsradius in medial-lateraler Richtung erstreckt. Damit ist der Clavicula-Abschnitt 11 der Knochenplatte 10 gut an die geschwungene S-Form einer Clavicula anlegbar. Der laterale Endabschnitt 13 umfasst ein laterales Ende, in welchem die Längsachse L₁ sich gerade in lateraler Richtung erstreckt.

Zudem weist der Clavicula-Abschnitt 11 eine Vielzahl von Öffnungen 12 auf, umfassend ein Langloch 121, die ungefähr auf der Längsachse L₁ angeordnet sind. Auf dem lateralen Ende des lateralen Endabschnitts 13 sind zudem jeweils zwei Löcher beidseitig der Längsachse L₁ angeordnet. Der Clavicula-Abschnitt 11 weist einen posterioren Rand 21, einen anterioren Rand 14, einen lateralen Rand 22 auf, der einen mittleren Punkt 23 aufweist, durch welchen die Längsachse L₁ verläuft.

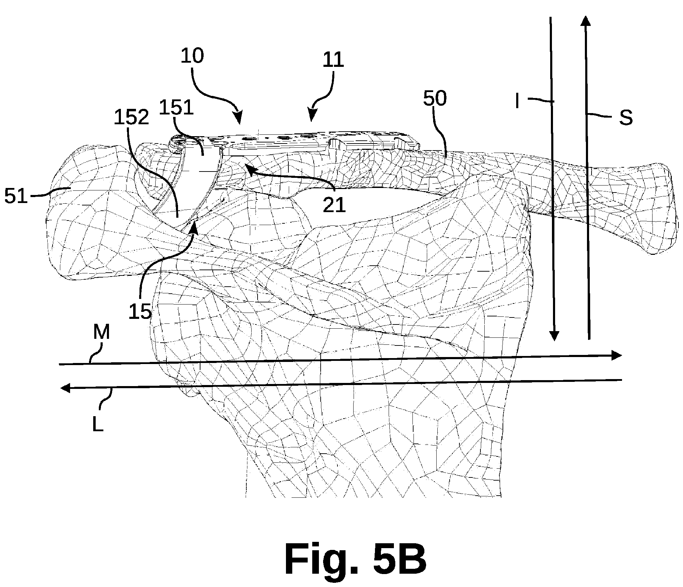

Der Zwischenabschnitt 15 erstreckt sich in posteriorer Richtung seitlich relativ zur Längsachse L₁ vom posterioren Rand 21 weg. Der Zwischenabschnitt 15 in Fig. 2 bildet einen geschwungenen tordierten Abschnitt, der in den Acromion-Abschnitt 16 übergeht. Der tordierte Abschnitt ist in einer medial-lateralen Richtung gekrümmt. Damit kann der Zwischenabschnitt 15 sich um ein laterales Ende der Clavicula erstrecken (siehe auch Fig. 5B). Der Zwischenabschnitt 15 verbindet den Acromion-Abschnitt 16 mit dem Clavicula-Abschnitt 11.

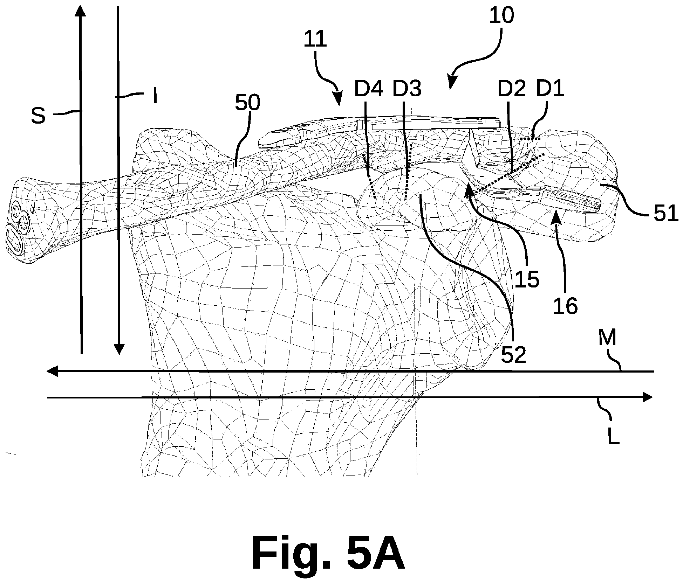

Der Acromion-Abschnitt 16 weist ein mediales Ende 19 auf, welches in dem Zwischenabschnitt 15 beginnt. Der Acromion-Abschnitt 16 erstreckt sich entlang einer kurvilinearen Längsachse L₂ vom medialen Ende 19 bis zu einem gegenüberliegenden lateralen Ende 20. Figur 2 zeigt zudem, dass ein minimaler Abstand a des Acromion-Abschnitts 16 von dem mittleren Punkt 23 des lateralen Rands 22 wenige Millimeter beträgt. Damit kann trotz einer Überbrückung des Acromio-Claviculargelenkspalts (siehe auch Fig 5A und 5B) der Knochenplatte, die den Spalt seitlich umgeht, das Acromion gut unterstützt werden.

In Fig. 2 liegt das laterale Ende 20 des Acromion-Abschnitts 16 in anterior-posteriorer Richtung in der gewählten Ansicht nahe einer gedachten Verlängerung der Längsachse L₁. Der Acromion-Abschnitt 16 ist vom medialen Ende 19 zum lateralen Ende 20 in posteriorer Richtung hin gekrümmt. Ein Winkel zwischen der ersten und zweiten Längsachse L₁, L₂ in Fig. 2 variiert über die Länge des Acromion-Abschnitts 16 in einem Bereich von 10° bis 35°. Somit kann das Acromion, welches lateral des Acromio-Claviculargelenkspalts angeordnet ist, von inferior gestützt werden.

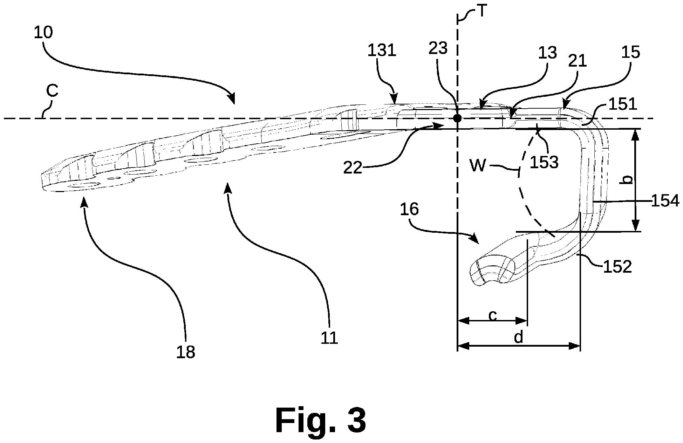

Figur 3 zeigt eine Draufsicht auf die erfindungsgemässe Knochenplatte 10 nach Fig. 1 etwa in lateral-medialer Richtung. Die Knochenplatte 10 ist so ausgerichtet, dass ein gerades Ende 131 des lateralen Endabschnitts 13 im Wesentlichen in der Bildebene angeordnet ist. Figur 3 zeigt zudem, dass dieses gerade Ende 131 im Wesentlichen parallel zu einer horizontalen Claviculaebene C ausgebildet ist. Damit kann die Knochenplatte 10 auf einer superioren Seite der Clavicula angelegt werden. Das mediale Ende 18 des Clavicula-Abschnitts 11 der Knochenplatte 10 ist angewinkelt zu der der Claviculaebene C angeordnet, in welcher der laterale Endabschnitt liegt.

Von einer Verbindungsstelle am posterioren Rand 21 des lateralen Endabschnitts 13 erstreckt sich der Zwischenabschnitt 15 zunächst seitlich weg, also in posteriorer Richtung. Der Zwischenabschnitt 15 weist gerade Bereiche 153, 154 und dazwischen liegende gebogene Bereiche 151, 152 auf.

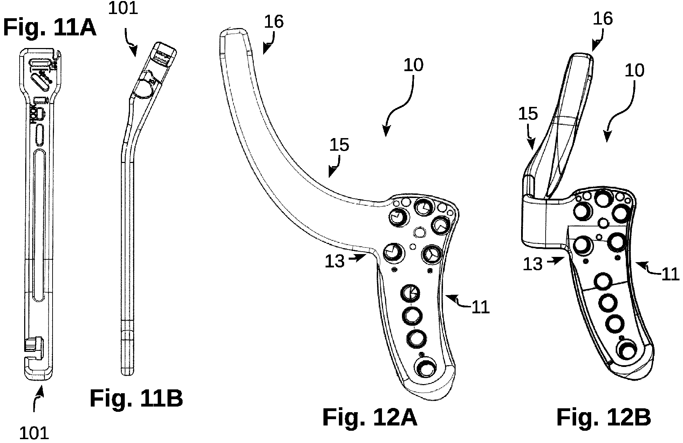

Der Zwischenabschnitt 15 kann durch Umformen in die in Fig. 3 gezeigte Form verbogen/tordiert werden (siehe Figs. 12A - 12B). Die zwei gebogenen Bereiche 151, 152 des Zwischenabschnitts 15 sind jeweils um etwa 90° gebogen, sodass der Zwischenabschnitt eine U-förmige Form annimmt. Der Zwischenabschnitt 15 ist somit über seine gesamte Länge in anterior-posteriorer Richtung um eine Winkel W von etwa 180° gebogen. Der Zwischenabschnitt 15 erstreckt sich bis zu dem Acromion-Abschnitt 16 zum Stützen des Acromion von inferior.

Ein Rohling der Knochenplatte 10 kann hingegen einen vollständig planar ausgebildeten Zwischenabschnitt 15 aufweisen (siehe Fig. 12A) .

Ein mittlerer Abstand d des Zwischenabschnitts 15 zum mittleren Punkt 23 des lateralen Rands 22 in anterior-posteriorer Richtung ist in einer lateralen Ansicht auf den lateralen Endabschnitt grösser als ein mittlerer Abstand c des Acromion-Abschnitts 16 zum mittleren Punkt 23. Damit kann der Acromio-Claviculargelenkspalt überbrückt werden, sodass der Zwischenabschnitt 15 und der Acromion-Abschnitt 16 ausserhalb des Acromio-Claviculargelenkspalts angeordnet werden können. Zudem wird ohne übermässige mechanische Interaktionen mit dem umliegenden Gewebe gleichzeitig eine zuverlässige Abstützung des Acromion von inferior ermöglicht. Um diese Abstände besser zu visualisieren, wurde in Fig. 3 eine senkrechte Transversalebene T eingezeichnet, die sich durch den mittleren Punkt 23 des lateralen Rands 22 erstreckt und senkrecht zur horizontalen Claviculaebene C ausgerichtet ist.

Ein Abstand b von einer Unterseite des Endabschnitts 13 zu einer Verbindungsstelle des Zwischenabschnitts 15 zum Acromion-Abschnitt 16 beträgt in einer Richtung senkrecht zum lateralen Endabschnitt etwa 5 - 20 mm, bevorzugt 9 - 16 mm. Der Abstand kann in Abhängigkeit der Physiologie des Patienten so gewählt sein, dass der Zwischenabschnitt 16 sich von der Höhe einer superioren Fläche der Clavicula bis zu der Höhe einer inferioren Fläche des Acromion erstreckt. Figur 3 zeigt zudem, dass der Acromion-Abschnitt 16 zumindest teilweise superior zu einem tiefsten Punkt des Zwischenabschnitts 15 angeordnet ist.

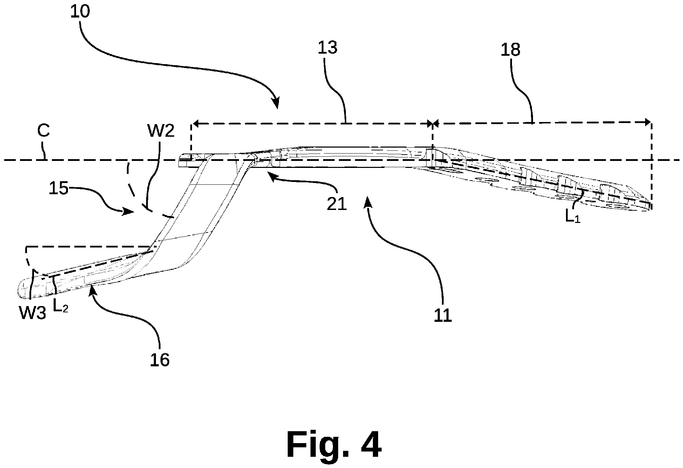

Figur 4 zeigt eine Seitenansicht der erfindungsgemässen Knochenplatte 10 nach Fig. 1 in posterior-anteriorer Richtung. Das mediale Ende 18 der Knochenplatte ist geneigt zu der horizontalen Claviculaebene C angeordnet und der laterale Endabschnitt 13 ist im Wesentlichen in der horizontalen Ebene C angeordnet. Die Längsachse L₁ (siehe Fig. 2) des lateralen Endabschnitts 13 liegt im Wesentlichen in der horizontalen Ebene C. Eine kurvilineare Längsachse L₁ des Clavicula-Abschnitts 11 weist einen Knick in superior-inferiorer Richtung zwischen dem medialen Ende und dem lateralen Endabschnitt 13 auf.

Der Zwischenabschnitt 15 erstreckt sich vom posterioren Rand 21 des Clavicula-Abschnitts 11 seitlich in posteriorer Richtung weg. Zudem erstreckt sich der Zwischenabschnitt 15 in lateraler Richtung in einem Winkel W2 von ca. 55° relativ zur horizontalen Ebene C / zur Längsachse L₁. Damit kann der Acromio-Claviculargelenkspalt überspannt werden, sodass der Acromion-Abschnitt 16 das Acromion zuverlässig von inferior in superiorer Richtung stützen kann.

Der Acromion-Abschnitt 16 erstreckt sich entlang einer Längsachse L₂, die in einem Winkel W3 von ca. 16° relativ zu der horizontalen Ebene C / der Längsachse L₁ angeordnet ist. Damit wird vom Acromion-Abschnitt 16 eine rampenartige Struktur gebildet, mit der das Acromion unterschiedlich stark, in Abhängigkeit der lateralen Position der Knochenplatte 10, auf der Clavicula, angehoben werden kann.

Figur 5A zeigt eine Seitenansicht der erfindungsgemässen Knochenplatte nach Fig. 1 in anteriorer Perspektive, die zur Behandlung einer Fraktur der Clavicula (und/oder von Bänderabrissen, nicht gezeigt in Fig. 5A) angebracht wurde. Der Clavicula-Abschnitt 11 der Knochenplatte 10 ist auf der Clavicula 50 angebracht und wurde durch Einbringen von Knochenschrauben in die Öffnungen auf der Clavicula 50 befestigt. Die Clavicula 50 weist eine Fraktur auf, die von der Knochenplatte 50 überspannt wird. Die Knochenplatte 10 ist beidseitig der Fraktur an der Clavicula 50 befestigt. Die Pfeile in Fig. 5A geben jeweils eine superiore Raumrichtung S, eine inferiore Raumrichtung I, eine laterale Raumrichtung L, und eine mediale Raumrichtung M an, die den Raumrichtungen einer ordnungsgemäss positionierten Knochenplatte 10 entsprechen.

Figur 5A zeigt zudem, dass sich der Zwischenabschnitt 15 seitlich in posteriorer Richtung vom Clavicula-Abschnitt 11 weg erstreckt und von der Clavicula teilweise verdeckt wird. Der Zwischenabschnitt 15 erstreckt sich teilweise um eine posteriore Aussenfläche der Clavicula 50 am lateralen Ende der Clavicula 50. Zudem erstreckt sich der Zwischenabschnitt 15 in lateraler Richtung in einer teilweise tordierten Form, sodass der sich anschliessende Acromion-Abschnitt 16 das Acromion 51 von inferior in superiorer Richtung stützt. Der Acromion-Abschnitt 16 erstreckt sich in einem Winkel zur horizontalen Claviculaebene C / zu der Längsachse L₁ des lateralen Endabschnitts 13, sodass eine rampenartige Struktur gebildet wird (siehe auch Fig. 4). Dadurch kann das Acromion 51 durch eine Wahl der lateralen Positionierung der Knochenplatte 10 relativ zur Clavicula 50 patientenspezifisch unterschiedlich stark angehoben werden.

Figur 5A zeigt zudem schematisch das Ligamentum acromio-claviculare D1, das Ligamentum coraco-acromiale D2, das Ligamentum trapezoideum D3, und das Ligamentum conoideum D4 exemplarisch als gestrichelte Linien. Das Ligamentum acromio-claviculare D1 erstreckt sich zwischen dem Coracoid 52 und dem Acromion 51. Das Ligamentum coraco-acromiale D2 erstreckt sich zwischen dem Acromion 51 und der Clavicula 50. Das Ligamentum trapezoideum D3 und das Ligamentum conoideum D4 erstrecken sich zwischen dem Coracoid 52 und der Clavicula 50. Figur 5A zeigt, dass die Knochenplatte 10 so geformt und gestaltet ist, dass sie bei ordnungsgemässer Platzierung nicht, oder nur geringfügig, mit den Bändern interagiert. Der Abriss eines oder mehrerer dieser Bänder kann mit der Knochenplatte 10 somit besonders schonend behandelt werden.

Figur 5B zeigt eine Seitenansicht der erfindungsgemässen Knochenplatte 10 in posteriorer Perspektive. Der Zwischenabschnitt 15 erstreckt sich seitlich vom posterioren Rand 21 weg. Durch zwei gebogene Bereiche 151, 152 des Zwischenabschnitts 15 (siehe Fig. 3), kann der Zwischenabschnitt 15 sich posterior enganliegend um die Clavicula 50 erstrecken. Die Biegung des Zwischenabschnitts 15 ist an eine äussere Kontur der posterioren Aussenfläche der Clavicula angepasst. Das Acromion 51 kann durch den Acromion-Abschnitt (nicht sichtbar in Fig. 5B) von inferior her in superior Richtung gestützt werden, während der Clavicula-Abschnitt 11 auf der superioren Oberfläche der Clavicula 50 angeordnet ist. Die Pfeile in Fig. 5B geben jeweils eine superiore Raumrichtung S, eine inferiore Raumrichtung I, eine laterale Raumrichtung L, und eine mediale Raumrichtung M an, die den Raumrichtungen einer ordnungsgemäss positionierten Knochenplatte 10 entsprechen.

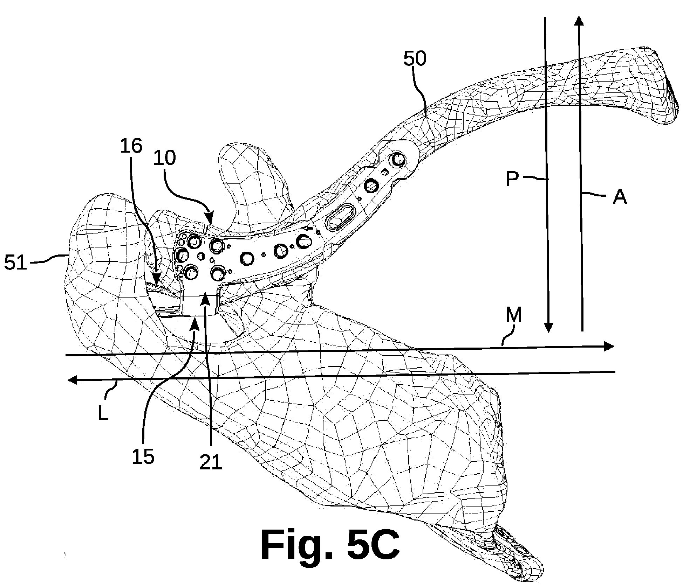

Figur 5C zeigt eine superiore Draufsicht der erfindungsgemässen Knochenplatte 10 nach Fig. 1, die zur Behandlung einer Fraktur der Clavicula 50 angebracht wurde. Die Pfeile in Fig. 5C geben jeweils eine anteriore Raumrichtung A, eine posteriore Raumrichtung P, eine laterale Raumrichtung L, und eine mediale Raumrichtung M an, die den Raumrichtungen einer ordnungsgemäss positionierten Knochenplatte 10 entsprechen.

Ein Zwischenabschnitt 15 der Knochenplatte 10 erstreckt sich von einem posterioren Rand 21 der Knochenplatte 10 seitlich weg. Der Zwischenabschnitt 15 erstreckt sich in posteriorer Raumrichtung P so weit, dass kein Bereich der Clavicula 50 in inferiorer Richtung unter dem Zwischenabschnitt 15 angeordnet ist. Daraufhin erstreckt sich der Zwischenabschnitt 15 entlang der inferioren Raumrichtung I und geht schliesslich in den Acromion-Abschnitt 16 über, der das Acromion 51 von inferior in superiorer Richtung stützt. Somit erstreckt sich der Zwischenabschnitt 15 um ein laterales Ende der Clavicula 50 seitlich herum, ohne im Acromio-Claviculargelenkspalt angeordnet oder an diesen angrenzenden Gelenkflächen zu beeinflussen / beeinträchtigen. Zudem kann die Knochenplatte 10 zum Positionieren in lateral-medialer Richtung L, M auf der Clavicula 50 bewegt werden, um die superiore-inferiore Ausrichtung des Acromions 51 und der Clavicula 50 zu optimieren.

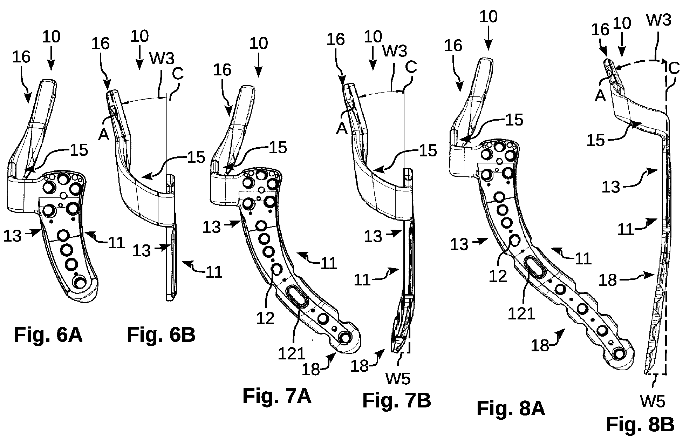

Die Figuren 6A und 6B zeigen eine Draufsicht und eine posteriore Seitenansicht einer zweiten, kurzen Ausführungsform der erfindungsgemässen Knochenplatte 10. Die Knochenplatte 10 weist einen Clavicula-Abschnitt 11 auf, der vollständig in einer planaren Fläche angeordnet ist. Zudem weist der Clavicula Abschnitt 11 lediglich eine Krümmung in medial-lateraler Richtung auf, die zu einem medialen Ende hin in anteriorer Richtung gekrümmt ist.

Figuren 7A und 7B zeigen eine Draufsicht und eine posteriore Seitenansicht einer dritten, mittellangen Ausführungsform der erfindungsgemässen Knochenplatte 10.

Figuren 8A und 8B zeigen eine Draufsicht und eine posteriore Seitenansicht einer vierten, langen Ausführungsform der erfindungsgemässen Knochenplatte 10.

Die Clavicula Abschnitte 11 der Ausführungsformen 7A bis 8B weisen in medial-lateraler Richtung zwei entgegengesetzte Krümmungen auf, wobei eine der Krümmungen zu einem medialen Ende 18 hin in posteriorer Richtung gekrümmt ist.

Das mediale Ende 18 der Ausführungsformen in Figs. 7A - 8B der Knochenplatte 10 ist jeweils in einem Winkel W5 von etwa 10° relativ zur Claviculaebene C angewinkelt. Zudem weisen diese Ausführungsformen jeweils eine Öffnung 12 auf, die als Langloch 121 ausgebildet ist.

Die Knochenplatten 10 der Figuren 6A - 8B weisen einen Clavicula-Abschnitt 11 mit einem lateralen Endabschnitt 13 auf, der in einer Claviculaebene C angeordnet ist. Der Zwischenabschnitt 15 in den Figuren 6A - 8B erstreckt sich von der Verbindungsstelle zum posterioren Rand der Knochenplatte 10 zunächst vollständig in der Claviculaebene C. Die Figuren 6A - 8B zeigen zudem, dass der Acromion-Abschnitt 16 in einer Acromialebene A liegt, welche in einen Winkel W3 von ca. 16° zu der Claviculaebene C geneigt ist.

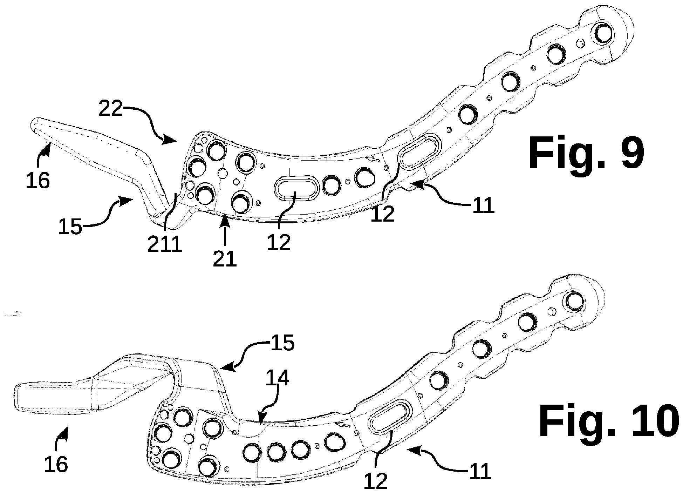

Figur 9 zeigt eine Draufsicht einer fünften erfindungsgemässen Ausführungsform einer Knochenplatte 10, bei der der Zwischenabschnitt 15 sich von einem Übergangsbereich 211 des posterioren Rands 21 seitlich weg erstreckt. Der Übergangsbereich 211 des posterioren Rands 21 bildet einen Übergang zum lateralen Rand 22 der Knochenplatte 10, also einen abgerundeten Bereich, der an den lateralen Rand 22 angrenzt. Ein Acromion-Abschnitt 16, der sich an den Zwischenabschnitt 15 anschliesst, sowie ein Clavicula-Abschnitt 11 ist ähnlich zu den zuvor beschriebenen Ausführungsformen ausgebildet.

Der Clavicula-Abschnitt 11 weist im Gegensatz zu den zuvor beschriebenen Ausführungsformen zusätzlich zu einer Vielzahl von Öffnungen 12 zur Befestigung von Knochenschrauben zwei Öffnungen 12 auf, die als Langlöcher ausgebildet sind. Einige der Öffnungen 12 sind zur Verblockung der Knochenschrauben in unterschiedlichen Orientierungen konfiguriert.

Figur 10 zeigt eine Draufsicht einer sechsten erfindungsgemässen Ausführungsform einer Knochenplatte 10, bei der sich der Zwischenabschnitt 15 vom anterioren Rand 14 seitlich vom Clavicula-Abschnitt 11 wegerstreckt. Im Übrigen entspricht diese Ausführhungsform und insbesondere die Ausgestaltung von Zwischenabschnitt 15 und Acromion-Abschnitt 16 im Wesentlichen der ersten Ausführungsform.

Figur 11A und 11B zeigen eine Draufsicht und Seitenansicht eines plattenspezifischen Biegewerkzeugs 101 zum manuellen Verbiegen und/oder Tordieren der Knochenplatte. Mittels zwei solcher Werkzeuge 101 kann eine Platte ergriffen und individuell an die Anatomie angebogen werden.

Die Figuren 12A und 12B zeigen eine Draufsicht einer erfindungsgemässen Knochenplatte 10 mit einem sich lateral erstreckenden Zwischenabschnitt 15 und Acromion-Abschnitt 16 vor und nach der Verbiegung/Tordierung durch Umformen.

Die Figur 12A zeigt einen Rohling der Knochenplatte 10. Der Zwischenabschnitt 15 und der Acromion-Abschnitt 16 der Knochenplatte 10 sind vor der Verbiegung/Tordierung im Wesentlichen in einer Ebene mit einem lateralen Endabschnitt 13 des Clavicula-Abschnitts 11 angeordnet. Ein mediales Ende des Clavicula-Abschnitts 11 kann ebenfalls in der Claviculaebene, i.e. nicht angewinkelt angeordnet sein. Ein solcher Rohling ermöglicht eine besonders einfache Fertigung der Knochenplatte 10, die anschliessend gemäss Fig. 12B durch Umformen gebogen/tordiert werden kann. Der Zwischenabschnitt 15 und der Acromion-Abschnitt 16 der Knochenplatte 10 erstrecken sich zuerst in posteriorer Richtung von einem posterioren Rand des Clavicula-Abschnitts 11 weg. Zudem erstrecken sich der Zwischenabschnitt 15 und Acromion-Abschnitt 16 von einer Verbindungsstelle am Clavicula-Abschnitt 11 in einem Bogen von etwa 80° in eine laterale Richtung relativ zur posterioren Richtung.

Die Knochenplatte 10 in Fig. 12A kann im Wesentlichen ausschliesslich durch eine Biegung/Tordierung mittels Umformens um in lateral-medialer Richtung verlaufende Biegekanten gebogen werden. Der gebogene Zwischenabschnitt 15 weist danach eine im Wesentlichen C-förmige Form auf (siehe Fig. 3). Der Acromion-Abschnitt 16 kann durch Umformen zudem so tordiert werden, dass der Acromion-Abschnitt geneigt zur Ebene des lateralen Endabschnitt 13 des Clavicula-Abschnitts 11 angeordnet ist (siehe Figs. 4 und 6A - 8B).

## Patentansprüche

1. Knochenplatte (10) zum Behandeln einer Fraktur einer Clavicula (50) und/oder von Abrissen der coraco-claviculären (D3, D4), coraco-acromialen (D2), und/oder acromio-claviculären (D1) Bänder, enthaltend:
- einen Clavicula-Abschnitt (11) zum Anlegen an die Clavicula (50), wobei der Clavicula-Abschnitt (11) einen lateralen Endabschnitt (13), einen medialen Endabschnitt (18), einen lateralen Rand (22), einen posterioren Rand (21), einen medialen Rand, einen anterioren Rand (14), eine superiore Oberfläche und eine inferiore Oberfläche, sowie mindestens eine Öffnung (12) zum Einsetzen eines Fixierelements, insbesondere einer Knochenschraube, aufweist,
- einen Acromion-Abschnitt (16) zum Anlegen an ein der Clavicula (50) benachbartes Acromion (51),
- einen Zwischenabschnitt (15), welcher sich vom Clavicula-Abschnitt (11) zum Acromion-Abschnitt (16) erstreckt,
**dadurch gekennzeichnet, dass** der Zwischenabschnitt (15) sich, insbesondere in superiorer Ansicht seitlich von einer Verbindungsstelle vom Clavicula-Abschnitt (11) weg erstreckt, wobei die Verbindungsstelle sich wenigstens teilweise am posterioren oder am anterioren Rand befindet, und/oder
dass die Form und Grösse des Zwischenabschnitts (15) und des Acromion-Abschnitts (16) derart gewählt ist, dass bei bestimmungsgemässer Anwendung der Zwischenabschnitt (15), und insbesondere alle Bestandteile der Knochenplatte (10), zumindest teilweise und bevorzugt vollständig ausserhalb des Acromio-Claviculargelenkspalts liegen, und/oder
die Form und Grösse des Zwischenabschnitts (15) derart gewählt ist, dass bei bestimmungsgemässer Anwendung mindestens ein Teil des Zwischenabschnitts (15) sich um eine posteriore oder um eine anteriore Seite am lateralen Ende der Clavicula herum erstreckt.

2. Knochenplatte (10) gemäss Anspruch 1, wobei die Form und Grösse des Zwischenabschnitts (15) und des Acromion-Abschnitts (16) derart gewählt ist, dass bei bestimmungsgemässer Anwendung der Acromion-Abschnitt (16) das Acromion von inferior in superior Richtung unterstützt und sich teilweise um das Acromion erstreckt.

3. Knochenplatte (10) gemäss einem der vorhergehenden Ansprüche, wobei sich der Zwischenabschnitt (15) vom lateralen Endabschnitt (13) des Clavicula-Abschnitts (11) zu einem medialen Ende (19) des Acromion-Abschnitts (16) erstreckt.

4. Knochenplatte (10) gemäss einem der vorhergehenden Ansprüche, wobei die Knochenplatte eine Längsachse (L1) aufweist, die sich vom medialen Endabschnitt (18) zum lateralen Endabschnitt (13) erstreckt und sich durch einen mittleren Punkt (23) des lateralen Randes (22) erstreckt,
wobei der Zwischenabschnitt (15) bezogen auf eine posterior-anteriore Richtung zumindest teilweise eine Krümmung aufweist, sodass insbesondere ein mittlerer Abstand (c) des Acromion-Abschnitts (16) von dem mittleren Punkt (23) in einer lateralen Ansicht in posterior-anteriorer Richtung kleiner ist als der mittlere Abstand (d) des Zwischenabschnitts (15) von dem mittleren Punkt (23).

5. Knochenplatte (10) gemäss einem der vorhergehenden Ansprüche, wobei der Acromion-Abschnitt (16) zumindest abschnittweise eine Krümmung aufweist, wobei die Krümmung sich in einer superioren Ansicht in anterior-posteriorer Richtung erstreckt und/oder in einer anterioren Ansicht in superior-inferiorer Richtung erstreckt.

6. Knochenplatte (10) gemäss einem der vorhergehenden Ansprüche, wobei eine Breite und/oder eine Dicke des Acromion-Abschnitts (16) sich ausgehend vom Zwischenabschnitt verändert, insbesondere kontinuierlich verändert, bevorzugt verjüngt oder verbreitert.

7. Knochenplatte (10) gemäss einem der vorhergehenden Ansprüche, wobei die Form und Grösse des Acromion-Abschnitts (16) und/oder des Zwischenabschnitts (15) derart gewählt ist, dass der Acromion-Abschnitt (16) und/oder der Zwischenabschnitt manuell mit einem plattenspezifischen Biegewerkzeug (101) biegbar und/oder um seine Längsachse (L₁) tordierbar ist.

8. Knochenplatte (10) gemäss einem der vorhergehenden Ansprüche, wobei der Acromion-Abschnitt (16) in einer lateralen Seitenansicht im Wesentlichen U-, C-, I-, J- oder Z-förmig ausgebildet ist.

9. Knochenplatte (10) gemäss einem der vorhergehenden Ansprüche, wobei der Zwischenabschnitt in einer lateralen Seitenansicht im Wesentlichen U-, C-, I-, J- oder Z-förmig ausgebildet ist.

10. Knochenplatte (10) gemäss einem der vorhergehenden Ansprüche, wobei der Acromion-Abschnitt (16) zum mittleren Punkt (23) des lateralen Rands (22) des Clavicula-Abschnitts (11) in einer senkrechten Ansicht auf den lateralen Endabschnitt einen minimalen Abstand (a) aufweist, der im Bereich von 0.1 mm bis 35 mm, insbesondere 1 mm bis 25 mm, vorzugsweise 1.5 mm bis 15 mm, liegt.

11. Knochenplatte (10) gemäss einem der vorhergehenden Ansprüche,
wobei der Acromion-Abschnitt (16) eine Längsachse (L₂) umfasst und ein laterales Ende (13) des Clavicula-Abschnitt (11) mindestens teilweise in einer Claviculaebene (C) liegt, und wobei die Claviculaebene (C) und die Längsachse (L₂) des Acromion-Abschnitts (16) in einer anterioren Ansicht unter einem Winkel oder Winkelbereich ausgewählt aus 0.1° bis 45°, insbesondere aus 3° bis 35°, vorzugsweise aus 6° bis 25°, zueinander verlaufen.

12. Knochenplatte (10) gemäss einem der vorhergehenden Ansprüche, wobei ein senkrecht zum Clavicula-Abschnitt (11) gemessene Abstand (b) des Acromion-Abschnitts (16) vom Clavicula-Abschnitt (11) im Bereich von 0.1 mm bis 30 mm, insbesondere in einem Bereich von 2 mm bis 25 mm, vorzugsweise in einem Bereich von 5 mm bis 18 mm, liegt.

13. Knochenplatte (10) gemäss einem der vorhergehenden Ansprüche, wobei der Clavicula-Abschnitt (11) vom lateralen Rand (22) zum medialen Rand in Richtung der Längsachse (L₁) eine erste Länge aufweist von 15 mm bis 180 mm aufweist, insbesondere von 80 mm bis 120 mm und/oder wobei der Acromion-Abschnitt (16) von einem medialen Ende (19) zu einem lateralen Ende (20) in Richtung der Längsachse (L₂) eine zweite Länge aufweist, von 5 mm bis 40 mm, insbesondere im Bereich von 10 mm bis 30 mm, vorzugsweise im Bereich von 15 mm bis 25 mm, aufweist.

14. Knochenplatte (10) gemäss einem der vorhergehenden Ansprüche, wobei die Längsachse (L₂) des Acromion-Abschnitts (16) in einer superioren Ansicht unter einem Winkel relativ zur ersten Längsachse (L₁) verläuft, wobei der Winkel im Bereich von -60° bis 60°, insbesondere -30° bis 30°, vorzugsweise -15° bis 15°, liegt.

15. Knochenplatte (10) gemäss einem der vorhergehenden Ansprüche, wobei der Zwischenabschnitt (15) zumindest einen gebogenen Bereich aufweist, sodass der Zwischenabschnitt (15) in einer lateralen Ansicht der Knochenplatte (10) über die gesamte Länge des Zwischenabschnitts (15) in anterior-posterior Richtung um einen Winkel (W) ausgewählt aus 90° bis 200°, insbesondere um 120° bis 190°, vorzugsweise um 160° bis 185° gebogen ist.

16. System umfassend eine Knochenplatte (10) nach einem der vorhergehenden Ansprüche und ein plattenspezifisches Instrument (101) zum manuellen Verbiegen und/oder Tordieren der Knochenplatte (10).

17. Verfahren zum Behandeln einer Fraktur einer Clavicula (50) und/oder von Abrissen der coraco-claviculären (D3, D4), coraco-acromialen (D2), und/oder acromio-claviculären (D1) Bänder mit einer Knochenplatte (10), insbesondere gemäss einem der vorangehenden Ansprüche, enthaltend die Schritte
a) Anlegen des Clavicula-Abschnitts (11) an eine Clavicula (50),
c) Anlegen des Acromion-Abschnitts (16) an ein der Clavicula (50) benachbartes Acromion (51), sodass das Acromion (51) von inferior in superiorer Richtung gestützt wird,
c) derart, dass die Knochenplatte (10) ausserhalb des Acromio-Claviculargelenkspalts liegt.

18. Verfahren nach Anspruch 17, wobei das Verfahren umfasst, dass die Knochenplatte (10) in lateral-medialer Richtung bewegt wird, um die superiore-inferiore Ausrichtung des Acromions (51) und der Clavicula (50) zueinander einzustellen.
